# EUROPEAN PATENT SPECIFICATION

(11) **EP 1 915 394 B1**
(45) Date of publication and mention of the grant of the patent: **27.01.2021**
(21) Application number: 06753745.6
(22) Date of filing: 19.05.2006
(51) Int. Cl.: C07K 16/00

(54) **HIGH-LEVEL EXPRESSION OF RECOMBINANT ANTIBODY IN A MAMMALIAN HOST CELL**
EXPRESSION AUF HOHEM NIVEAU VON REKOMBINANTEM ANTIKÖRPER IN EINER SÄUGETIER-WIRTSZELLE
EXPRESSION DE NIVEAU ELEVE D'UN ANTICORPS RECOMBINANT D'UNE CELLULE HOTE DE MAMMIFERE

(30) Priority: 20.05.2005 GB 0510277; 03.06.2005 GB 0511353; 28.12.2005 GB 0526372
(43) Date of publication of application: 30.04.2008
(73) Proprietor: Lonza Biologics plc., Berkshire, Slough SL1 4DY (GB)
(72) Inventor: Rance, James, London N21 3JX (GB); Kalwy, Stephan, Slough SL1 3RW (GB); Young, Robert, London NW2 1SR (GB); Gay, Robert, Bondi Junction NSW 2022 (AU)
(74) Representative: Eder, Michael
(86) International application number: PCT/EP2006/004767
(87) International publication number: WO 2006/122822

(56) References cited:
- WO-A2-03/014344
- WO-A2-2005/075514
- NORDERHAUG L ET AL: "Versatile vectors for transient and stable expression of recombinant antibody molecules in mammalian cells" JOURNAL OF IMMUNOLOGICAL METHODS, ELSEVIER SCIENCE PUBLISHERS B.V.,AMSTERDAM, NL, vol. 204, no. 1, 12 May 1997 (1997-05-12), pages 77-87, XP004107719 ISSN: 0022-1759
- WHITTLE N ET AL: "EXPRESSION IN COS CELLS OF A MOUSE - HUMAN CHIMAERIC B72.3 ANTIBODY" PROTEIN ENGINEERING, OXFORD UNIVERSITY PRESS, SURREY, GB, vol. 1, no. 6, 1 December 1987 (1987-12-01), pages 499-505, XP000005250 ISSN: 0269-2139
- WELLS K D ET AL: "CODON OPTIMIZATION, GENETIC INSULATION, AND AN RTTA REPORTER IMPROVE PERFORMANCE OF THE TETRACYCLINE SWITCH", TRANSGENIC RESEARCH, LONDON, GB, vol. 8, no. 5, 1 January 1999 (1999-01-01) , pages 371-381, XP009000915, ISSN: 0962-8819, DOI: 10.1023/A:1008952302539

## Description

The present invention relates to a mammalian host cell with a high-level expression of recombinant antibody wherein the host cell contains a double-gene vector with gene-optimized nucleotide sequences coding for the light and the heavy antibody chains, the double-gene vector itself and a process for enhancing the production of recombinant antibody in a mammalian host cell.

Antibodies, also called immunoglobulins, are glycoproteins, which specifically recognise foreign molecules called antigens. When foreign antigens invade humans or other animals, an immunological response is triggered which involves the production of antibodies by B-lymphocytes. By this immunological response, micro-organisms, viruses and bacterial toxins can be rendered harmless. In vertebrates five immunoglobulin classes with different functions in the immune system are known: IgG, IgM, IgA, IgD and IgE. They have a basic structure of two identical heavy chain polypeptides and two identical light chain polypeptides. The heavy and light chains are held together by disulfide bridges and non-covalent interactions. The chains themselves comprise variable and constant domains. The variable domains of the heavy and light chain are more variable in amino acid sequence than the constant region domains and are located at the N-terminal part of the antibody molecule. The heavy and light chain variable domains each contain three stretches of hypervariable or complementary determining region (CDR) sequence which together form the unique antigen-recognition site.

Several functional antigen-binding fragments can be engineered by proteolysis of antibodies, for example by papain digestion, pepsin digestion or other enzymatic approaches, yielding, for example, Fab, F(ab')₂ or Fv fragments. The development of recombinant DNA technology led to the design of several new antibodies and antibody fragments. For example the functionalities of these proteins have been altered resulting in novel and improved functions. Thus, it is possible to reduce unwanted immunological properties in medical applications. Also, smaller recombinant antibody fragments may be expressed having the advantage over whole antibodies in applications requiring tissue penetration and rapid clearance from the blood.

Engineered antibody molecules and their fragments are being increasingly exploited as scientific and clinical tools for therapy and diagnosis of diseases.

The unique ability of antibodies to specifically recognize and bind with high affinity to virtually any type of antigen, make them attractive as starting point for novel biopharmaceutical products and scientific research. More applications outside research and medicine can be considered, such as consumer applications. Examples are the use of antibodies in shampoos to prevent the formation of dandruff or in toothpaste to prevent caries and to protect against tooth decay caused by caries, respectively. Other conceivable applications include for example the use in biosensors, treatment of wastewater, industrial scale separation processes or as abzymes, i.e. the use of an artificially created antibody as an enzyme. A completely different use of the binding capacity of antibody fragments is in the design of fusion proteins. For example the 3' end of the heavy chain constant region gene is fused to the 5' end of an enzyme gene. If the antibody has specificity for a tumour antigen, for example, then the fusion can be used to deliver a toxin or toxic enzyme to a tumour cell in order to selectively kill it. Alternatively a chelate (capable of binding a radio-isotope) can be chemically coupled to recombinant anti-tumour antibody or antibody fragment, also selectively killing tumour cells. In human medicine these approaches are sometimes referred to as the "Magic bullet".

However, for these purposes large quantities of antibodies are required. Several expression systems are available, both from prokaryotic and eukaryotic origin. The choice of system depends on many factors, including the molecular species being expressed and the precise sequence of the individual antibody. One of the most widely used expression systems for recombinant proteins including antibodies is the Chinese Hamster ovary (CHO) cell mammalian expression system. It is one of the few cell types allowing for simple and efficient high-density suspension batch culture of animal cells. CHO cells allow for very high product yields and are comparatively robust to metabolic stresses. By expression of recombinant proteins in CHO cells it is possible to achieve a glycosylation pattern which is similar but not identical to that obtained from human cells.

Recently, the yield of recombinant antibodies in CHO cells has been greatly enhanced by optimization of the culture medium composition, bioreactor design and parameters of cell cultivation. Thus, huge progress has been made for achieving high density growth, high specific productivity and prolonged viability of cultured cells. Further, yield enhancement at the transcriptional level has been widely optimized, using strongest possible promoters and enhancer elements of mostly viral origin. Further, comparative studies on vector systems and host cells showed that the use of tandem vectors, in which both of the heavy and light gene transcription units were inserted, resulted in much higher antibody production levels compared with the use of a mixture of each heavy chain vector and light chain vector.

However, despite refined genetic expression technology apparent levels of antibody expression can still vary up to 200-fold. According to Bentley et al., Hybridoma, 17 (1998), 559-567, each antibody is expressed with a characteristic efficiency, determined by a combination of factors including the level of light chain synthesis and heavy and light chain "compatibility". According to Strutzenberger et al., J. Biotechnol., 69 (1999), 215-226, a high expression rate of light chain is beneficial in increasing secretion rates of whole antibody. In a CHO cell-based expression system they showed that reduction of light chain expression led to a low intracellular concentration of light chain and accumulation of heavy chain in the endoplasmic reticulum due to retention by chaperones. Borth et al., J. Biotechnol., 8 (1999), 57-66, analysed the content of intracellular light and heavy chain mRNA and their respective polypeptides and the specific secretion rates of three sub-clones with low, medium and high specific production rates. For the three sub-clones a correlation between intracellular content in light chain and the secretion rate was found, while the intracellular content in heavy chain was the same for all three sub-clones. The authors conclude that the assembly in the endoplasmic reticulum is one of the major rate limiting factors in antibody production. Smales et al., Biotechnol. Bioeng., 88(4) (2004), 474-488, describe that in GS-NSO cell lines producing recombinant monoclonal antibody the intracellular light chain content is significantly in excess of the intracellular heavy content. They conclude that the intracellular molar ratio was approximately 10:1.

Thus, it is entirely possible that along the antibody production pathway a considerable portion of light and heavy chains are not properly assembled into tetrameric immunoglobulin. First of all, there are assembly problems of the monomeric protein chains; even in the presence of 70% free IgG light chain, considerable amounts of monomeric IgG heavy chain may be detectable. Excess light chain may also be shed to the cell culture medium along with functional, fully assembled monoclonal antibody (mAb). The simple chemical law of mass action does not apply in a cell. Efforts to elevate selectively the expression level of the heavy chain have not proven successful. Gass et al., Trends Immunol. 25(1) (2004), 17-24, suggest that in plasma cells, which are natural antibody secreting B-cells, the best achievable mAb titre is reached in the presence of excess light chain.

Likely explanations are also inefficient assembly and/or premature selective degradation of the heavy chain by targeting to the proteosome; quality control and degradations timing are known to involve glucose tagging of the carbohydrate moieties. Protein domain assembly may be driven by affinity interactions, formation of disulfide bonds bringing domains into close proximity and/or the need to bury exposed hydrophobic patches on the surface of individual domains; in the initial stage of assembly and folding, it is believed that such patches are shielded by chaperone proteins. It is now well-known that secretory glycoprotein is folded and assembled into higher order complexes in the cell's inner endoplasmic reticulum (ER) compartment of the cell shortly after protein synthesis. The ER is the sole compartment to comprise specific auxiliary assembly factors along with quality control mechanism (Ellgaard et al., Quality control in the secretory pathway, Science. 1999 Dec 3;286:1882-8; Helenius et al., Intracellular functions of N-linked glycans, Science. 2001 Mar 23;291:2364-9). Once the point of successful assembly has passed, no further assembly will take place as passage through the secretory pathway of the cell proceeds. In fact, some cell types such as CHO cells secrete unassembled chains of both types whereas others such as NSO cells retain selectively non-assembled chains of the IgG heavy type only and probably target them to degradative pathways.

Previous studies have correlated changes in specific recombinant monoclonal antibody production with cellular abundance of secretory pathway proteins that occur as a consequence of changes in either cell environment (Lambert and Merten, Biotechnol. Bioeng., 54(2) (1997), 165-180) or time in culture (Downham et al., Biotechnol. Bioeng., 51(6) (1996), 691-696). Thus, overexpression of ER chaperones and foldases has been used to engineer the rate of recombinant antibody secretion by eukaryotic cells. For example, WO 03/057897 teaches a method for expressing a recombinant protein comprising co-expression of chaperone proteins and small heat shock proteins. Those additional proteins are said to promote successful folding and assembly and thus the portion of correctly folded, most active product protein.

However, overexpression of individual chaperones and foldase has not increased production of monoclonal antibody in mammalian cells. For example, it was shown that BIP overexpression in mammalian cells reduced the secretion of proteins it associates with (Domer and Kaufman, Biologicals, 22(2) (1994), 103-112) and that overexpression of PDI in CHO cells decreased the secretion of a disulfide-rich fusion protein ((Davis et al., Biotechnol. Prog., 16(5) (2000), 736-743. Thus, the co-expression of several auxiliary factors may decrease total expression rate of the protein product and requires careful optimization of individual co-expression rates of such auxiliary factors. Different product protein might dependent to a varying degree on individual, only partially overlapping chaperone functions of which a multitude have become known to date, e.g. GroEL, GroES, DnaK, DnaJ, GrpE, ClpB, IbpA, Ibp. Thus it seems, that is not desirable or possible to co-express all of them at a time at the sole expense of product protein production rate.

The technical problem underlying the present invention is to avoid the disadvantages of the prior art and to provide novel means and processes for enhancing antibody production in a mammalian host cell which allows the high level production of standard, tetrameric whole IgG antibody having Fc receptor activity and consisting of at least two different polypeptide chains.

The present invention solves this technical problem by providing a mammalian expression vector for Chinese Hamster Ovary host cells comprising at least a first transcription unit containing a first nucleotide sequence encoding the light chain of an antibody and a second transcription unit containing a second nucleotide sequence encoding the heavy chain of an antibody, characterized in that the codon composition of both the first and second nucleotide sequence is adapted to the codon bias of Chinese Hamster Ovary host cells.

Also described herein is a mammalian expression vector comprising at least a first transcription unit containing a first synthetic nucleotide sequence encoding a first polypeptide chain and a second transcription unit containing a second synthetic nucleotide sequence encoding a second polypeptide chain wherein the first and second synthetic nucleotide sequences are based on naturally occurring nucleotide sequences and wherein the first and second polypeptide chain can form a molecule comprising at least one or multiple copies of each of the first and the second polypeptide chain, characterized in that the codon composition of both the first and second nucleotide sequence is adapted to the codon bias of the genes of a given mammalian host species which is different from the mammalian species from which the naturally occurring nucleotide sequences originally were derived.

Thus, the present invention provides a mammalian expression vector with nucleotide sequences adapted to the codon bias of CHO cells, i.e. gene-optimized nucleotide sequences. The inventors of the present invention have tested the inventive double-gene vector comprising gene-optimized genes of the light and heavy chains in the mammalian CHO cell expression system, wherein the amino acid sequences of the light and heavy chains were not altered by the gene optimization. Surprisingly and unexpectedly it was found that the use of a vector in which both heavy and light chain genes had been gene-optimized significantly raised the median level of antibody production from 37.8 µg/ml to 51.3 µg/ml. In contrast, expressing a gene-optimized heavy chain alone in combination with a non-optimized light chain was not sufficient to enhance the antibody expression level. Thus, by use of the inventive mammalian double-gene expression vector comprising gene-optimized genes for both the heavy and light chains it is possible to achieve a markedly increased total yield of secreted antibody in comparison to control vectors containing one gene-optimized gene and one non-optimized gene or control vectors in which both heavy and light chain genes are not gene-optimized. The markedly enhanced level of antibody production obtained by the vector with both gene-optimized heavy and light chain genes has to be attributed to the gene-optimized heavy and light chain genes.

Further results obtained by the inventors of the present invention indicate that it is advantageous to optimize the whole coding gene sequences and not only certain parts thereof. For example, if only the N-termini of the light and heavy chain genes were gene-optimized than the overall antibody production was not or only slightly increased.

The results obtained by the inventors of the present invention sharply contrast the observations described in the prior art. From the prior art it is known that there is a correlation between the intracellular content of the light chain and the specific secretion rate of antibody which suggests that the light chain must be expressed in excess compared to the heavy chain to achieve a high level antibody production and if not so, heavy chain will be accumulated in the endoplasmic reticulum due to retention by chaperones. In contrast, the results of the inventors rather suggest that also in mammalian expression systems light chain expression and heavy chain expression have to be balanced to achieve a high level production of secreted antibody. Not wishing to be bound by any particular theory, it is believed that for achieving a high level production of antibody it is not sufficient to have only an increased intracellular pool of light chain. It appears that also a high intracellular pool of heavy chain is necessary.

Furthermore the results of the present inventors show that not only the assembly in the endoplasmic reticulum is one of the major rate limiting factors in antibody production but also the regulation of transcription and the regulation of mRNA stability. Since the gene optimization used by the inventors did not alter the amino acid sequences of heavy and light chains the higher antibody production cannot be attributed to an altered assembly of the chains or an altered ER-associated degradation of the misfolded or unfolded polypeptide chains. The increased antibody production obtained by the double-gene vector in which both the heavy chain and light chain genes had been gene-optimized rather suggests that the transcriptional efficiency and mRNA stability were optimized due to removal of cis-acting sequence motifs such as internal TAT-boxes, chi-sites, repeat sequences, cryptic splice sites etc leading to an increased translational efficiency of the respective genes.

Thus, the present invention solves the underlying technical problem by providing a mammalian expression vector for Chinese Hamster Ovary host cells comprising at least a first transcription unit containing a first nucleotide sequence encoding the light chain of an antibody and a second transcription unit containing a second nucleotide sequence encoding the heavy chain of an antibody, characterized in that the codon composition of both the first and second nucleotide sequence is adapted to the codon bias of Chinese Hamster Ovary host cells.

Also described herein is a mammalian expression vector comprising at least a first transcription unit containing a first synthetic nucleotide sequence encoding a first polypeptide chain and a second transcription unit containing a second synthetic nucleotide sequence encoding a second polypeptide chain wherein the first and second synthetic nucleotide sequences are based on naturally occurring nucleotide sequences and wherein the first and second polypeptide chain can form a molecule comprising at least one or multiple copies of each of the first and the second polypeptide chain, characterized in that the codon composition of both the first and second nucleotide sequences is amended such that their mRNAs are translated with higher efficiency.

In the context of the present disclosure "a molecule comprising at least one or multiple copies of each of the first and the second polypeptide chain" is a multisubunit molecule, in particular a secretory multisubunit molecule. The molecule can consist of one or more copies of each of two different polypeptide chains. The molecule can comprise more than two different polypeptide chains. In a preferred embodiment the molecule is a secretory antibody. As described herein, the molecule can be another protein consisting of different polypeptide chains or subunit. Examples for such multisubunit proteins include, without being restricted to, a multisubunit enzyme, a receptor molecule and an ion-channel.

In the context of the present invention a "mammalian expression vector" is a, preferably isolated and purified, DNA molecule which upon transfection into an appropriate mammalian host cell provides for a high level expression of both polypeptide chains within the host cell.

The mammalian expression vector according to present invention comprises at least two separate transcription units. An expression vector with two separate transcription units is also referred as double-gene vector. In the double gene as claimed the first synthetic nucleotide sequence or first gene of the first transcription unit encodes the light chain of an antibody and the second synthetic nucleotide sequence of the second transcription unit encodes the heavy chain of an antibody. Another example described herein is a double-gene vector, in which the two synthetic nucleotide sequences encode two different subunits of a protein such as an enzyme.

However, it is also possible that the inventive expression vector comprises more than two separate transcription units, for example three, four or even more separate transcription units each of which comprises a different synthetic nucleotide sequence encoding a different polypeptide chain. Described herein is a vector with four separate transcription units, each of which contains a different synthetic nucleotide sequence encoding one subunit of an enzyme consisting of four different subunits.

In the context of the present invention the term "gene optimization" means a technique by which a plurality of alterations are introduced into a natural nucleotide sequence such as a gene of interest resulting in the generation of a new synthetic nucleotide sequence. In the context of the present invention "synthetic nucleotide sequence" therefore means a nucleotide sequence which is derived by gene optimization from a natural nucleotide sequence. The natural nucleotide sequence can be a genomic sequence or a cDNA obtained from a genomic sequence and lacking intron sequences.

The aim of the gene optimization is to increase the translational efficiency of the gene and to ensure its optimal expression in a target organism or in a tissue or a cell of this target organism whereby the target organism is different from the organism from which the nucleotide sequence was originally derived. Based on the different abundance of different degenerate transfer RNAs in different species each organism has its preferred choice of codon usage. Proteins that exhibit in a given organism or species the highest level of expression have a high codon bias (i.e., the extent to which the same codons for amino acids tend to be utilized in a gene).

Gene optimization therefore includes the replacement of at least one existing codon of the natural nucleotide sequence with a synonymous codon preferably used in the target organism. The synonymous codon corresponds to an iso-tRNA which, when compared to the iso-tRNA corresponding to the replaced codon, is in higher abundance in the target cell or organism.

In the present invention the codon composition of the first and second nucleotide sequence is adapted to the codon bias of genes of CHO host cells.

As described herein, the codon composition of the first and second nucleotide sequence is adapted to the codon bias of Homo sapiens genes.

Eukaryotic gene expression is a complex mechanism that can be regulated on the transcriptional, post-transcriptional, translational and post-translational levels. Experiments on yeast have shown that 80% of the proteome is expressed during exponential growth and that 85% of the ribosomes are engaged in translation suggesting that mRNAs have to compete for ribosomes. Studies of the present inventors on NSO cell lines producing a chimeric antibody have further revealed that recombinant mRNA accounts for approx. 20% of total mRNA. This suggests that the demand for ribosomes exceeds the cellular resources and therefore ribosome shortfall can impede cell maintenance and growth.

Regulation of mRNA stability is an important component of the regulation of gene expression. The structural characteristics of individual mRNAs can influence the translation process. In higher eukaryotes it has been shown that for example the length of the leader, the presence of secondary structure either upstream or downstream of the initiation codon and the length of the poly (A) tail can affect the efficiency of mRNA translation. Stable secondary structures within a leader can impede scanning of the 40S ribosomal subunit in its search for the initiation codon and thereby inhibit translation. It is known that consensus cis-acting sequences and sequence motifs exist that are involved in determining mRNA stability. Cooperative interactions of elements in nucleic acids are also involved in restricting expression of cellular genes on the post-transcriptional levels. Such inhibitory sequences (INS) are active within mRNAs. Further, several viral and cellular mRNAs have evolved regulatory elements, i.e. internal ribosome entry site (IRES) elements within the leader that function through cap-independent mechanisms to promote 40S ribosomal subunit binding internally to an mRNA.

Gene optimization also includes alterations which improve the transcriptional efficiency of a synthetic nucleotide sequence and confer on the mRNA an enhanced stability and/or increase the translational efficiency of the mRNA.

Thus, gene optimization includes alterations in the GC content or GC distribution in one or both of the synthetic nucleotide sequences compared to the natural nucleotide sequences. Thus, the disclosure relates to a mammalian expression vector in which the two synthetic nucleotide sequences have a GC content and/or GC distribution which are different from that of the corresponding naturally occurring nucleotide sequences. A "different" GC content means that the synthetic nucleotide sequence can have a higher or a lower GC content than the naturally occurring nucleotide sequence depending on the target mammalian host cell. In particular in one or both of the synthetic nucleotide sequences the GC content of the 5'UTR is reduced in comparison to the corresponding naturally occurring nucleotide sequences. Further in one or both of the synthetic nucleotide sequences the GC content the 3'UTR is increased in comparison to the corresponding naturally occurring nucleotide sequences.

In another aspect, one or both of the two synthetic nucleotide sequences have an AT content and/or AT distribution that is different from that of the corresponding naturally occurring nucleotide sequences. In particular in one or both of the synthetic nucleotide sequences the AT content of the 3'UTR is reduced in comparison to the corresponding naturally occurring nucleotide sequences.

In still another aspect in one or both of the synthetic nucleotide sequences the length of the 3'UTR and/or the 5'UTR is altered in comparison to the corresponding naturally occurring nucleotide sequences In particular, the length of the 3'UTR is increased. In particular, the length of the 5'UTR is adjusted to approx. 60 bp.

In another preferred embodiment one or both of the two synthetic nucleotide sequences contain less cis-acting sequence motifs than the corresponding naturally occurring nucleotide sequences. "Less cis-acting sequence motifs" means, that the naturally occurring nucleotide sequence has at least one more cis-acting sequence motif compared to the synthetic nucleotide sequence. Preferably, the synthetic nucleotide sequence has less than 95%, less than 90%, less than 80%, less than 70%, less than 60%, less than 50%, less than 40%, less than 30%, less than 20% or even less than 10% of all the cis-acting sequence motifs present in the naturally occurring nucleotide sequence. Most preferred, the synthetic nucleotide sequence has no cis-acting sequence motifs at all. It is preferred that gene optimization includes in particular alterations of cis-acting sequence motifs that are present in 5'UTR and 3'UTR.

"cis-acting sequence motifs" or "cis-acting sequence elements" include without being restricted to, internal TATA boxes, chi-sites, ribosomal entry sites such as IRES sites, AT-rich or GC-rich sequence stretches, ARE, INS or CRS sequence elements, repeat sequences which can affect RNA stability, cryptic slice donor and acceptor sites etc. Thus, the two synthetic nucleotide sequences contain preferably less internal TATA-boxes, chi-sites and/or ribosomal entry sites in comparison to the naturally occurring nucleotide sequence. The two synthetic nucleotide sequences can also contain less ARE, INS and/or CRS sequence elements in comparison to the naturally occurring nucleotide sequence. Further, the two synthetic nucleotide sequences can contain less repeated sequences in comparison to the naturally occurring nucleotide sequence, so that their RNA will form less secondary structures than the naturally occurring nucleotide sequences. Also, the two synthetic nucleotide sequences can contain less cryptic splice donor and acceptor sites in comparison to the naturally occurring nucleotide sequence.

In an embodiment of the present invention gene optimization includes only alterations of a particular type of cis-acting sequence motifs such as alteration and/or elimination of ARE elements present in the naturally occurring nucleotide sequence. In another embodiment gene optimization includes alteration and/or elimination of two or more types of cis-acting sequence motifs present in the naturally occurring nucleotide sequence, for example elimination of TATA-boxes and ARE sequence elements and cryptic splice donor and acceptor sites. In still another embodiment gene optimization includes alteration and/or elimination of all conceivable types of cis-acting sequence motifs. Gene optimization of the first and second synthetic nucleotide sequences can include alteration and/or elimination of different cis-acting sequence motifs.

Gene optimization includes alterations which remove alternative initiation sites such as uCDSs and uAUGs. The present disclosure therefore also relates to mammalian expression vectors wherein one or both of the two synthetic nucleotide sequences contain less alternative initiation sites than the corresponding naturally occurring nucleotide sequences.

In one embodiment of the present invention the whole nucleotide sequence encoding a an antibody chain is gene-optimized. In a further embodiment only certain regions of the nucleotide sequence are gene-optimized. An example for this is the gene optimization of the N-terminus of the nucleotide sequence. According to the invention it is of course possible that the first and second synthetic nucleotide sequences are the result of different gene optimizations. For example it is possible in that in one of the two synthetic nucleotide sequences the entire sequence is gene-optimized whereas in the other synthetic sequence only a part of the sequence is gene-optimized. It is, however also possible, that in the synthetic nucleotide sequences different features are altered. For example in one of the synthetic nucleotide sequences only the GC content was altered whereas in the other synthetic nucleotide sequence only certain cis-acting elements were removed.

In a particularly preferred embodiment of the invention the gene optimization does not result in an altered amino acid sequences, i.e. the first and second antibody chains encoded by the synthetic nucleotide sequences have the same amino acid sequences compared to the corresponding antibody chains encoded by the naturally occurring nucleotide sequences.

However, according to the invention it is of course possible that the gene optimization will lead to an altered amino acid sequence of one or both antibody chains encoded by the synthetic nucleotide sequences; i.e. either the first antibody chain encoded by the first synthetic nucleotide sequence or the second antibody chain encoded by the second synthetic nucleotide sequence or both of them have different amino acid sequences compared to the corresponding antibody chains encoded by the naturally occurring nucleotide sequences. A preferred example relates to antibody chains which have less glycosylation sites or amended glycosylation sites compared to the corresponding antibody chains encoded by the naturally occurring nucleotide sequences.

The invention relates to a mammalian expression vector, in which the first synthetic nucleotide sequence encodes the light chain of an antibody and the second synthetic nucleotide sequence encodes the heavy chain of an antibody. Preferably, upon expression the first and second polypeptide chain can form an antibody or immunoglobulin. In another preferred embodiment the first and/or second synthetic nucleotide sequence is fused to the gene of an effector protein. In this case upon expression the first and second polypeptide chain can form a fusion protein, in which an effector protein is coupled to an antibody or immunoglobulin.

An immunoglobulin according to the present invention can have Fc-receptor activity or complement activation activity or both. Whereas complement activation is clearly defined in the art as to relate to induction of the complement cascade (by possibly different pathways), Fc-receptor activity in the context of the present invention is to be understood as to the activation of cellular Fc receptors which trigger a cellular response, e.g. in the case of naturally occurring IgG or IgA triggered phagocytic or cytotoxic activities or e.g. in the case of release of mast cell granules upon triggering of cellular receptors by natural IgE class immunoglobulin. Similarly, amongst natural antibodies, both IgM and IgG class antibodies may trigger complement activation. It goes without saying that any such effector activities may vary amongst naturally occurring subclasses of antibodies and their known allotypes, and accordingly may vary amongst the antibodies of the present invention. In the context of the present invention, however, it is possible that Fc-receptor activity or complement activation effector domains are engineered into any given immunoglobulin structure by means of domain swapping, effectively transferring or adding the respective effector properties in such resulting immunoglobulin.

The immunoglobulin may be a naturally occurring type of immunoglobulin, apart from its specific binding for a given antigen, or it may be an engineered, artificial type of immunoglobulin. This includes species-chimeric antibodies or CDR grafted antibodies, antibodies created by gene shuffling or site-directed engineering, antibodies chemically modified with PEG or radioisotope-chelating moieties or fusion proteins linking an immunoglobulin moiety having afore said activity to any other proteinaceous moiety such as another enzymatically active domain. The extent to which every activity is conferred by a given immunoglobulin may vary. Both types of effector function are caused by the constant portion regions of the immunoglobulin heavy chain; for instance, the different human IgG subclasses vary in their relative efficacy to activate and amplify the steps of the complement cascade. In general, human IgG1 and IgG3 most effectively fix complement, IgG2 is less effective, and IgG4 does not activate complement. Assay formats to test for either of afore said activities are well-known to immunologists and other persons; suitable protocols may e.g. be found in standard immunochemistry lab manuals such as Harlow et al., Antibodies - a laboratory manual, Cold Spring Harbor Laboratory Press 1988. In naturally occurring immunoglobulin for instance, light chains have a single constant region domain and heavy chains have several constant region domains. - All human subclasses IgG1, IgG2, IgG3 and IgG4 mediate cytotoxic effector functions through constant chain portions (ADCC: antibody directed cytotoxicity), brought about by interaction of the antibody with killer cells/cytotoxic T-lymphocytes; this is quite notable because IgG4 has often been said not to mediate such effects. However, it has been found that consistingly, human IgG4 is intrinsically capable of mediating ADCC whilst its extent is strongly modulated/dependent on the source of effector cells used in the assays such as ⁵¹Cr-release, due to a distinct natural polymorphism in humans at least. This has been shown by Greenwood J, Clark M, Waldmann H.: Structural motifs involved in human IgG antibody effector functions Eur J Immunol 1993; 5: 1098-1104.

The naturally occurring antibody or immunoglobulin classes IgG and IgA naturally have three constant region domains, designated CH1, CH2 and CH3, and the IgM and IgE classes have four constant region domains. In contrast, e.g. WO02/056910 devises artificial antibodies for human therapy that are devoid of the CH1 domain; such antibodies are encompassed by the notion of immunoglobulin according to the present invention as well.

Preferably, the immunoglobulin or Ig molecule comprises at least a hinge domain, a CH2 and a CH3 domain or functional variants thereof. Those domains form the essential Fc part e.g. in natural IgG. Detailed descriptions and definitions of these structural elements of an immunoglobulin are set forth in Amzel et al., Three-dimensional structure of immunoglobulins, Ann. Rev. Biochem. 48, 961-997 (1979); Davies et al., Structural basis of antibody function, Ann. Rev. Immunol. 1, 87-117 (1983); Hunkapiller et al., Diversity of immunoglobulin gene superfamily, Adv. Immunol. 44, 1-63 (1989). Said domains can be naturally occurring domains, artificially created chimeric versions of such domains or chimeric assemblies of such domains or versions engineered e.g. by site-directed mutagenesis. In the past, chimeric, CDR grafted mouse human chimeric antibodies were often used; alike, potential glycosylation sites in the variable or CH1/CL domain portions were often eliminated by site directed mutagenesis. Of course, the extent of engineering of any part of the immunoglobulin according to the present invention may be often limited by the need to avoid creating extended, strongly immunogenic motifs in engineered antibody, apart from the natural variability inherent to the complementarity determining regions. Apart from this, for the antigen-binding moiety upstream of the hinge portion that is conventionally coined the Fv portion (comprising the V_{H} and V_{L} domains) of e.g. IgG type antibody, the only requirement according to the present invention is that such portion is made up from two distinct polypeptide chains (when secreted) and has some antigen-binding property. It is possible that an immunoglobulin according to the present invention has increased antigen-binding valency achieved by multiplied variable domains arranged in a 'pearl-on-a-string' fashion in the antigen-binding Fv format.

In a particularly preferred embodiment of the invention the first nucleotide sequence encoding the light chain of an antibody comprises the sequence depicted in SEQ ID No. 3 and the second nucleotide sequence encoding the heavy chain of an antibody comprises the sequence depicted in SEQ ID No. 1. The amino acid sequence of the light chain encoded by SEQ ID No. 3 is depicted in SEQ ID No. 4. The amino acid sequence of the heavy chain encoded by SEQ ID No. 1 is depicted in SEQ ID No. 2.

In addition to the nucleotide sequences coding for the light and heavy chains the mammalian expression vector comprises regulatory DNA sequences that are required for an efficient transcription of mRNAs from the coding sequence and an efficient translation of the mRNAs in the host cell line. When the regulatory sequences are operably linked to the nucleotide sequence they will mediate the initiation of transcription of this nucleotide sequence and promote efficient protein synthesis from the corresponding mRNA within the environment of a mammalian cell. In a preferred embodiment of the present invention in each of the two transcription units the nucleotide sequences encoding the light and heavy chains are under the control of the same regulatory units.

Preferably, the mammalian expression vectors of the present invention further contain at least one expressible marker selectable in animal cells. Therefore, in a preferred embodiment the present mammalian expression vector comprises a third transcription unit encoding a selectable marker. Any selection marker commonly employed such as thymidine kinase (tk), dihydrofolate reductase (DHFR) or glutamine synthetase (GS) may be used. In a preferred embodiment, an expressible GS selection marker is employed (Bebbington et al., 1992, High-level expression of a recombinant antibody from myeloma cells using a glutamine synthetase gene as an amplifiable selectable marker, Bio/Technology 10:169-175; Cockett et al., 1990, High level expression of tissue inhibitor of metalloproteinases in Chinese Hamster Ovary (CHO) cells using Glutamine synthetase gene amplification, Bio/Technology 8: 662-667). The GS-system is one of only two systems that are of particular importance for the production of therapeutic proteins. In comparison to the dihydrofolate reductase (DHFR) system, the GS system offers a large time advantage during development because highly productive cell lines can often be created from the initial transfectant thus avoiding the need for multiple rounds of selection in the presence of increasing concentrations of selective agent in order to achieve gene amplification (Brown et al., 1992, Process development for the production of recombinant antibodies using the glutamine synthetase (GS) system, Cytotechnology 9:231-236).

Where used in particular for transient/episomal expression only, the expression vectors of the invention may further comprise an origin of replication such as origin of Epstein Barr Virus (EBV) or SV40 virus for autonomous replication/episomal maintenance in eukaryotic host cells but may be devoid of a selectable marker. Expression vectors of the invention can be for example, without being limited to, linear DNA fragments, DNA fragments encompassing nuclear targeting sequences or may be specially optimized for interaction with transfection reagents, animal viruses or suitable plasmids that can be shuttled and produced in bacteria.

The present invention solves the underlying technical problem also by providing a CHO host cell containing the mammalian expression vector of the present invention.

Other host cells described herein include be any vertebrate host cell line that can be, in contrast to primary cell lines, stably propagated in cell culture. Possible cell lines are e.g. COS cells, NSO cells, BHK cells, Sf-9 cells, 293 or 293-EBNA cells,

HBK-11 cells (ATCC-CRL12569; also see US6,136,599), Per-C6 cells (Crucell B.V., Netherlands; WO97/00326, also see EP-1161548) or HT1080 cells. For instance, HT1080 cells can be ordered as ATCC No. CCL-121 at the Amercian Type Culture Collection, Manassas/VA, U.S.A.. HT1080 cells have been found to allow of enhanced product glycosylation when used in combination with glutamine synthetase selection marker system (WO 03/064630).

According to the invention the mammalian host cell is a Chinese hamster ovary (CHO) cell or cell line (Puck et al., 1958, J. Exp. Med. 108: 945-955), in particular CHO-K1 cells (ATCC CCL-61), CHO pro3-, CHO DG44, CHO P12, the dhfr- CHO cell line DUK-BII (Chassin et al., PNAS 77, 1980, 4216-4220), DUXB11 (Simonsen et al., PNAS 80, 1983, 2495-2499) or CHO cells adapted for growth in serum-free suspension culture (i.e. excluding microcarrier-borne culture).

Described herein are host cells which are lymphoid cells, such as mammalian lymphoid cells, encompassing e.g. hybridoma, myeloma and trioma cells lines. Examples are e.g. non-secreting hybridoma such as SP2/0 and non-secreting myeloma cells e.g. such as NSO cell line ECACC No. 85110503 (European Collection of Cell cultures, Centre for Applied microbiology, Salisbury/Wiltshire SP4 0JG, United Kingdom) from mouse or YB2/3.0 Ag20 (described in GB2070313) from rat. Myeloma cells such as NSO cells truly are B-lymphoid cell types, namely plasmacytoma cell lines, although being routinely addressed in the art as 'myelomas' (Barnes et al., Cytotechnology 32:109-123, 2000).

Other examples of mammalian host cells include MRC5 human fibroblasts, 983M human melanoma cells, MDCK canine kidney cells, RF cultured rat lung fibroblasts isolated from Sprague-Dawley rats, B16BL6 murine melanoma cells, P815 murine mastocytoma cells and MT1A2 murine mammary adenocarcinoma cells.

For introducing the expression vector into an mammalian host cell according to the present invention any transfection technique such as those well-known in the art, e.g. electoporation, calcium phosphate co-precipitation, DEAE-dextran transfection, lipofection, can be employed if appropriate for a given host cell type. It is to be noted that the mammalian host cell transfected with the vector of the present invention is to be construed as being a transiently or stably transfected cell line. Thus, according to the present invention the present mammalian expression vector can be maintained episomally or can be stably integrated in the genome of the mammalian host cell.

A transient transfection is characterised by non-appliance of any selection pressure for a vector borne selection marker. A pool or batch of cells originating from a transient transfection is a pooled cell population that comprises cells which have taken up and do express and cells that have not taken up the foreign DNA. In transient expression experiments which commonly last 20-50 hours post transfection, the transfected vectors are maintained as episomal elements and are not yet integrated into the genome. That is the transfected DNA, does not usually integrate into the host cell genome. The host cells tend to lose the transfected DNA and overgrow transfected cells in the population upon culture of the transiently transfected cell pool. Therefore expression is strongest in the period immediately following transfection and decreases with time. Preferably, a transient transfectant according to the present invention is understood as a cell that is maintained in cell culture in the absence of selection pressure up to a time of 90 hours post transfection.

In a preferred embodiment of the invention the CHO host cell is stably transfected with the mammalian expression vector of the invention. Stable transfection means that newly introduced foreign DNA such as vector DNA is becoming incorporated into genomic DNA, usually by random, non-homologous recombination events. The copy number of the vector DNA and concomitantly the amount of the gene product can be increased by selecting for cell lines in which the vector sequences have been amplified after integration into the DNA of the host cell. Therefore, it is possible that such stable integration gives rise, upon exposure to further selection pressure for gene amplification, to double minute chromosomes in CHO cells. Furthermore, in case of a vector sequence, a stable transfection may result in loss of vector sequence parts not directly related to expression of the recombinant gene product, such as e.g. bacterial copy number control regions rendered superfluous upon genomic integration. Therefore, a transfected host cell has integrated at least part or different parts of the expression vector into the genome.

The present invention solves the underlying technical problem also by providing a process for improving the level of production of a secreted antibody, comprising a light chain and a heavy chain, comprising the steps of
a) transfecting a CHO host cell with an expression vector according to present invention encoding the light chain and the heavy chain,
b) culturing the host cell under appropriate conditions to enable propagation of the cell and expression and assembly of the two chains within the cell to form a molecule and secretion of molecule formed and
c) harvesting the antibody formed.

The process for increasing the level of production of a secreted antibody, is based on the use of the inventive mammalian double-gene expression vector comprising two different gene-optimized genes encoding the heavy and light chains of an antibody, which upon expression form the molecule desired. By the use of the inventive vector it is possible to achieve a markedly increased total yield of secreted antibody in comparison to control vectors containing only one gene-optimized gene or control vectors in which both genes are not gene-optimized. Thus, the inventive process advantageously results in a considerably enhanced level of production of the secreted antibody.

Preferably, in the inventive process both different antibody chains are expressed in a nearly 1:1 ratio. This is particularly advantageous if molecule desired is an antibody consisting of multiple copies of a heavy and a light chain. However, it is also possible to adjust the expression of the two antibody chains to another ratio. This can be achieved by applying different gene optimization strategies to the two different synthetic nucleotide sequences encoding the antibody chains or by operably linking the two synthetic nucleotide sequences with a different promoter.

As described herein the process can be used to increase the yield of any secreted molecule consisting of at least two different polypeptide chain. Examples for such multisubunit proteins include, without being restricted to an enzyme comprising several different subunits, a receptor molecule and an ion-channel.

Suitable media and culture methods for mammalian cell lines are well-known in the art, as described in US 5,633,162 for instance. Examples of standard cell culture media for laboratory flask or low density cell culture and being adapted to the needs of particular cell types include, without being restricted to, Roswell Park Memorial Institute (RPMI) 1640 medium (Morre, G., The Journal of the American Medical Association, 199, p.519 f. 1967), L-15 medium (Leibovitz, A. et al., Amer. J. of Hygiene, 78, lp.173 ff, 1963), Dulbecco's modified Eagle's medium (DMEM), Eagle's minimal essential medium (MEM), Ham's F12 medium (Ham, R. et al., Proc. Natl. Acad. Sc.53, p288 ff. 1965) or Iscoves' modified DMEM lacking albumin, transferrin and lecithin (Iscoves et al., J. Exp. med. 1, p. 923 ff., 1978). For instance, Ham's F10 or F12 media were specially designed for CHO cell culture. Other media specially adapted to CHO cell culture are described in EP-481 791. It is known that such culture media can be supplemented with fetal bovine serum (FBS, also called fetal calf serum FCS), the latter providing a natural source of a plethora of hormones and growth factors. The cell culture of mammalian cells is nowadays a routine operation well-described in scientific textbooks and manuals, it is covered in detail e.g. in R. Ian Fresney, Culture of Animal cells, a manual, 4th edition, Wiley-Liss/N.Y., 2000.

In a preferred embodiment of the present invention the cell culture medium used is devoid of fetal calf serum (FCS or FBS), which then is being termed 'serum-free'. Cells in serum-free medium generally require insulin and transferrin in a serum-free medium for optimal growth. Transferrin may at least partially be substituted by non-peptide chelating agents or siderophores such as tropolone as described in WO 94/02592 or increased levels of a source of an organic iron favorably in conjunction with antioxidants such as vitamin C. Most cell lines require one or more of synthetic growth factors (comprising recombinant polypeptides), including e.g. epidermal growth factor (EGF), fibroblast growth factor (FGF), insulin like growth factors I and II (IGFI, IGFII), etc.. Other classes of factors which may be necessary include: prostaglandins, transport and binding proteins (e.g. ceruloplasmin, high and low density lipoproteins, bovine serum albumin (BSA)), hormones, including steroid-hormones, and fatty acids. Polypeptide factor testing is best done in a stepwise fashion testing new polypeptide factors in the presence of those found to be growth stimulatory. Those growth factors are synthetic or recombinant. There a several methodological approaches well-known in animal cell culture, an exemplary being described in the following. The initial step is to obtain conditions where the cells will survive and/or grow slowly for 3-6 days after transfer from serum-supplemented culture medium. In most cell types, this is at least in part a function of inoculum density. Once the optimal hormone/growth factor/polypeptide supplement is found, the inoculum density required for survival will decrease.

In another preferred embodiment, the cell culture medium is protein-free, that is free both of fetal serum and individual protein growth factor supplements or other protein such as recombinant transferrin.

In another embodiment the process of the present invention includes a high-density growth of the host cells e.g. in an industrial fed-batch bioreactor. Conventional downstream processing may then be applied. Consequently, a high-density growth culture medium has to be employed. Such high-density growth media can usually be supplemented with nutrients such as all amino acids, energy sources such as glucose in the range given above, inorganic salts, vitamins, trace elements (defined as inorganic compounds usually present at final concentrations in the micromolar range), buffers, the four nucleosides or their corresponding nucleotides, antioxidants such as glutathione (reduced), vitamin C and other components such as important membrane lipids, e.g. cholesterol or phosphatidylcholine or lipid precursors, e.g. choline or inositol. A high-density medium will be enriched in most or all of these compounds, and will, except for the inorganic salts based on which the osmolarity of the essentially isotonic medium is regulated, comprise them in higher amounts (fortified) than the afore mentioned standard media as can be incurred from GB2251 249 in comparison with RPMI 1640. Preferably, a high-density culture medium according to the present invention is fortified in that all amino acids except for tryptophan are in excess of 75 mg/l culture medium. Preferably, in conjunction with the general amino acid requirement, glutamine and/or asparagine are in excess of 1 g/l, more preferably of 2 g/l of high-density culture medium. In the context of the present invention, high-density cell culture is defined as a population of animal cells having temporarily a density of viable cells of at least or in excess of 10⁵ cells/ml, preferably of at least or in excess of 10⁶ cells/ml, and which population has been continuously grown from a single cell or inoculum of lower viable cell density in a cell culture medium in a constant or increasing culture volume.

In a further preferred embodiment the process of the present invention includes a fed-batch culture. A fed-batch culture is a culture system wherein at least glutamine, optionally with one or several other amino acids, preferably glycine, is fed to the cell culture as described in GB2251249 for maintaining their concentration in the medium, apart from controlling glucose concentration by separate feed. More preferably, the feed of glutamine and optionally one or several other amino acids is combined with feeding one or more energy sources such as glucose to the cell culture as described in EP-229 809-A. Feed is usually initiated at 25-60 hours after start of the culture; for instance, it is useful to start feed when cells have reached a density of about 10⁶ cells/ml. It is well known in the art that in cultured animal cells, 'glutaminolysis' (McKeehan et al., 1984, Glutaminolysis in animal cells,in: Carbohydrate Metabolism in Cultured Cells, ed. M.J. Morgan, Plenum Press, New York, pp. 11-150) may become an important source of energy during growth phase. The total glutamine and/or asparagine feed (for substitution of glutamine by asparagine, see Kurano, N. et al., 1990, J. Biotechnology 15, 113-128) is usually in the range from 0.5 to 10 g per 1, preferably from 1 to 2 g per 1 culture volume; other amino acids that can be present in the feed are from 10 to 300 mg total feed per litre of culture, in particular glycine, lysine, arginine, valine, isoleucine and leucine are usually fed at higher amounts of at least 150 to 200 mg as compared to the other amino acids. The feed can be added as shot-addition or as continuously pumped feed, preferably the feed is almost continuously pumped into the bioreactor. It goes without saying that the pH is carefully controlled during fed-batch cultivation in a bioreactor at an approximately physiological pH optimal for a given cell line by addition of base or buffer. When glucose is used as an energy source the total glucose feed is usually from 1 to 10, preferably from 3 to 6 grams per litre of the culture. Apart from inclusion of amino acids, the feed preferably comprises a low amount of choline in the range of 5 to 20 mg per litre of culture. More preferably, such feed of choline is combined with supplementation of ethanolamine essentially as described in US 6,048,728, in particular in combination with feeding glutamine. It goes without saying that upon use of the GS-marker system, lower amounts of glutamine will be required as compared to a non-GS expression system since accumulation of excessive glutamine in addition to the endogenously produced would give rise to ammonia production and concomitant toxicity. For GS, glutamine in the medium or feed is mostly substituted by its equivalents and/or precursors, that is asparagine and/or glutamate.

Methods for harvesting, i.e. isolating and/or purifying a given protein from a cell, a cell culture or the medium in which the cells had been cultured are well known in the art. Proteins can be isolated and/or purified from biological material for example by fractionated precipitation with salts or organic solvents, ion exchange chromatography, gel chromatography, HPLC, affinity chromatography etc..

The Figures show:
- Fig. 1: Map of vector pcB72.3 comprising the genomic sequence of the heavy chain
- Fig. 2: Map of vector pcB72.3 HC cDNA comprising the cDNA of the heavy chain
- Fig. 3: Maps of vector pcB72.3 Geneart HC comprising a gene-optimized gene encoding the heavy chain under the control of the hCMV-MIE promoter and vector pcB72.3 Geneart HC/LC comprising a gene-optimized gene encoding the heavy chain and a gene-optimized gene encoding the light chain, wherein each of the genes is under the control of the hCMV-MIE promoter.
- Fig. 4: Relative antibody expression levels in transfected CHO-KISV cells. Comparison between vector pcB72.3 comprising genomic DNA encoding the heavy chain and vector pcB72.3 HC cDNA comprising cDNA encoding the heavy chain.
- Fig. 5: Relative antibody expression levels in transfected CHO-K1 SV cells. Comparison between vector pcB72.3 HC cDNA comprising cDNA encoding the heavy chain, vector pcB72.3 HC comprising a gene-optimized gene encoding the heavy chain and vector pcB72.3 HC/LC comprising a gene-optimized gene encoding the heavy chain and a gene-optimized gene encoding the light chain.

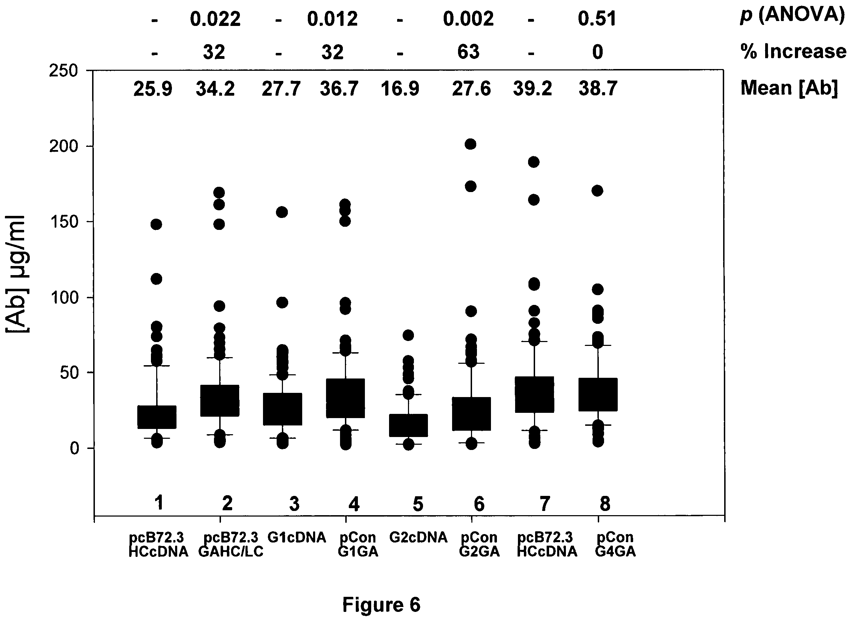

- Fig. 6: The Figure shows the effect of gene optimized constant regions on antibody production in transfected CHO-K1 SV cells. The vector pConG1GA contains the IgGI gene sequences with optimized constant regions and non-optimized variable regions. The vector pConG2GA contains the IgG2 gene sequences with optimized constant regions and non-optimized variable regions. The vector pConG4GA contains the IgG4 gene sequences with optimized constant regions and non-optimized variable regions. The vector GlcDNA contains non-optimized IgG1 gene sequences, the vector G2cDNA contains non-optimized IgG2 gene sequences and the vector pcB72.3 HCcDNA contains non-optimized IgG4 sequences. The vector pcB72.3 GAHC/LC, a vector with gene-optimized constant regions and gene optimized variable regions, was used as a control. The data for pcB72.3 (7) and pConG4GA (8) was obtained from a separate experiment, explaining the differences in the control values observed between pcB72.3 (1) and pcB72.3 (7).

The sequence listing shows:
SEQ ID No.1 shows the nucleic acid sequence encoding the optimized cB72.3 heavy chain.
SEQ ID No. 2 shows the amino acid sequence of the optimized cB72.3 heavy chain encoded by the nucleic acid sequence of SEQ ID No. 1.
SEQ ID No. 3 shows the nucleic acid sequence encoding the optimized cB72.3 light chain.
SEQ ID No. 4 shows the amino acid sequence of the optimized cB72.3 light chain encoded by the nucleic acid sequence of SEQ ID No. 3.

It is understood that the explanations and references made to a given preferred embodiment in the present specification of the invention relate likewise to all further preferred embodiment of the present invention.

The present invention is explained in more detail by the following examples.

### Examples

### Materials and Methods

### Cells used

CHO cell line CHOK1SV: is a variant of the cell line CHO-K1 and has been adapted to growth in suspension and protein-free medium.

### Propagation of CHOK1SV cells:

CHOK1SV cells were routinely propagated in suspension shaker flasks in CD-CHO medium (Invitrogen) supplemented with 6 mM L-glutamine. Seed concentration was 2 x 10⁵ cells/ml, and cells are split every 4 days. Flasks were gassed with 5% CO₂ and incubated at 36.5°C (between 35.5°C and 37.0°C) with orbital shaking at 140 rpm.

### Transient Transfection:

Transient transfections were performed using suspension-growing cells. Cells were counted and distributed onto wells of a 24-well plate at 2.5 x 10⁵ viable cells per well in a DMEM-based medium supplemented with 10% serum and 6 mM L-glutamine, and incubated overnight at +36.5 °C. The following day, the conditioned medium was replaced with 1 mL of fresh medium (as above) and the cells incubated for 3 hours at +37 °C.

For each transfection, 5 µg of each of the SGVs (HC and LC-SGVs mixed together) or 5 µg of the DGVs were re-suspended in 100 µL transfection medium (OptiMEM, Invitrogen). For positive controls, cells were also transfected with the vector pcB72.3, which encodes heavy chain and light chain genes for an IgG₄/kappa antibody which serves as a model antibody. A negative control (water only) was also included.

For each transfection, 5 µL of Lipofectamine-2000 reagent (Invitrogen) was diluted in 100 µL transfection medium, mixed and left to stand for 5 minute at room temperature. The DNA and diluted Lipofectamine reagent were combined, mixed and further left to stand at ambient temperature for 20 minutes. This 200 µL mixture was then added to a well of the 24-well plate containing the cells, and the cells were incubated for 4 or 10 days at +37 °C. The culture supernatant was collected and clarified by centrifugation prior to assay for presence of antibody by assembly ELISA.

### Stable transfections:

Cells used for transfections were grown in cell suspension culture, as detailed before. Cells from growing cultures were centrifuged and washed once in serum-free medium prior to being re-suspended to a concentration of 1.43 x 10⁷ cells/mL. A 0.7 mL volume of the cell suspension and 40 µg of plasmid DNA were added to an electroporation cuvette. The cuvette was then placed in the electroporation apparatus and a single pulse of 250 V and 400 µF was delivered. Following transfection, the cells were distributed into 96-well plates at approximately 2,500 host cells/well (5 x 10⁴/mL), using the non-selective DMEM-based medium supplemented with 10% dFCS. The plates were incubated at 36.5°C (between 35.5°C and 37.0°C) in an atmosphere of 10% CO₂ in air.

The day after the transfection, DMEM-based medium supplemented with 10% dFCS/66 µM L-methionine sulphoximine was added to each well (150 µL/well) to give a final L-methionine sulphoximine concentration of 50 µM. The plates were monitored to determine when the non-transfected cells died and when foci of transfected cells appeared. Foci of transfected cells became apparent approximately three to four weeks after transfection. All the cell lines examined and progressed further came from wells containing only a single colony.

### Assessment of Productivity of Cell Lines in Static Culture

The 96-well transfection plates were incubated for approximately three weeks to allow colony formation. The resulting colonies were examined microscopically to verify that the colonies were of a suitable size for assay (covering greater than 60% of the bottom of the well), and that only one colony was present in each well.

Suitable colonies were transferred to wells of 24-well plates containing 1 mL of selective growth medium (DMEM-based medium/10% dFCS/25µM L-methionine sulphoximine). These cultures were incubated for 14 days at 36.5°C (between 35.5°C and 37.0°C) in an atmosphere of 10% CO₂ in air. The supernatant of each well was harvested and analysed for the concentration of antibody present by the protein-A HLPC method.

### Assembly ELISA:

The antibody concentration of samples was determined using a sandwich ELISA which measures assembled human IgG. This involved capture of samples and standard onto a 96 well plate coated with an anti-human Fc antibody. Bound antibody is revealed with an anti-human light chain linked to horseradish peroxidase and the chromogenic substrate TMB. Colour development was proportional to the concentration of antibody present in the sample when compared to the standard.

### Protein A HPLC:

The Protein A affinity chromatography method for the measurement of IgG was performed on an Agilent 1100 HPLC. IgG product binds selectively to a Poros Protein A immunodetection column. Non-bound material is washed from the column and the remaining bound antibody is released by decreasing the pH of the solvent. The elution was monitored by absorbance at 280 nm and product was quantified (using Chemstation software) against a generic antibody standard and a correction is made for differences in extinction coefficients.

### Vector Construction

### a) Heavy Chain cDNA

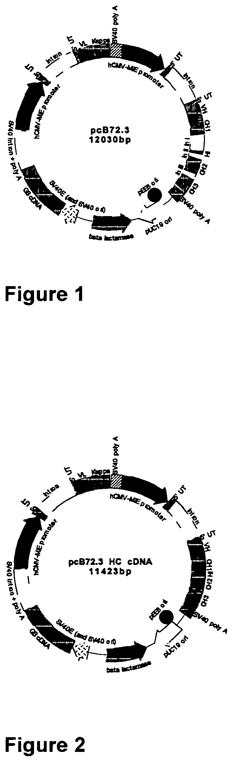

The HC cDNA version of pcB72.3 was generated by transiently transfecting the pcB72.3 expression vector (depicted in Figure 1) into CHOK1SV cells using Lipofecatmine-2000 (Invitrogen) according to the manufacturer's instructions. The following day, total RNA was extracted form the transfected cells as used as a template in cDNA synthesis. The cB72.3 HC sequence was amplified from the cDNA using specific primers. As this sequence is derived from the mRNA, it lacks the intron sequences. This sequence was then cloned into the vector pEE6.4, and combined with the vector pConK+VL to generate the pcB72.3 HC cDNA vector (depicted in Figure 2).

### b) Gene-optimized Genes

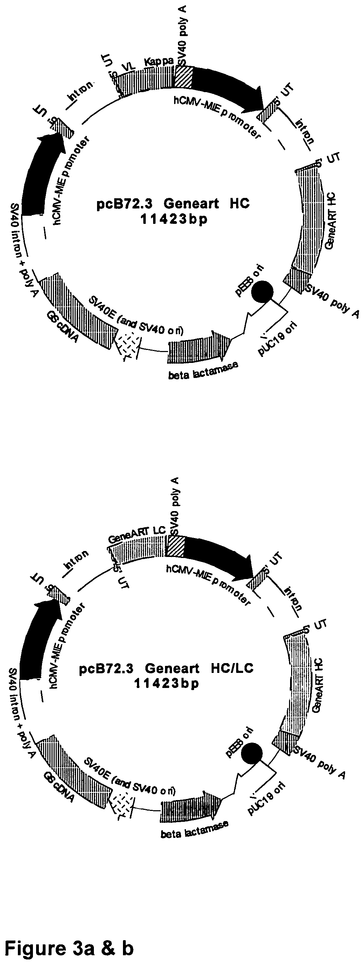

Gene optimization was run on cDNA sequences encoding the HC and LC of cB72.3. Optimisation was run on the sequence to remove all potential negatively acting sequences, and to optimise the codon usage. Then the optimal sequences were synthesized using a gene assembly approach. Following assembly, the HC and LC encoding sequences depicted in SEQ ID Nos. 1 and 3, respectively, were then cloned into vector pEE6.4 (for the HC) and vector pEE12.4 (for the LC) via *Hind* III and *EcoR* I sites. A DGV was then generated by combined the HC and LC expression cassettes by cloning via *Not* I and *Pvu* I restriction enzyme sites. This generated vectors pcB72.3 Geneart HC and pcB72.3 Geneart HC/LC depicted in Figure 3.

### c) Preparation of DNA for transfection

For the cDNA and Geneart vectors, a bulk preparation of DNA was generated using a Qiagen Maxiprep kit. For all transfections the vector DNA was linearised by digestion with *Pvu* I. Digested plasmid was examined by running a sample on an agarose gel to confirm complete linearization. The DNA was purified by phenol:chloroform extraction and aliquoted into lots of 40 µg. The DNA within each aliquot was precipitated by addition of 0.1 volumes 3 M sodium acetate, pH 5.2 and two volumes of ice cold 100% ethanol and stored at -20 ± 5 °C until required.

### Transfection, selection and overgrow cultures

CHOK1SV cells were transfected with the vector constructs using a standard electroporation method. Cells were plated out across 96-well plates. The following day, selective medium was added at 50 µM.

Plates were screened for developing colonies at 4 weeks post-transfection. For each transfection pool, approximately 100 colonies of appropriate size were transferred to 24-well plates in medium containing 25 µM MSX. cells were allowed to overgrow for 2 weeks, after which the cell culture medium from each well was collected and assessed by Protein A HPLC for the level of cB72.3 antibody present.

### Results and Discussions

### Genomic versus cDNA heavy chain sequence

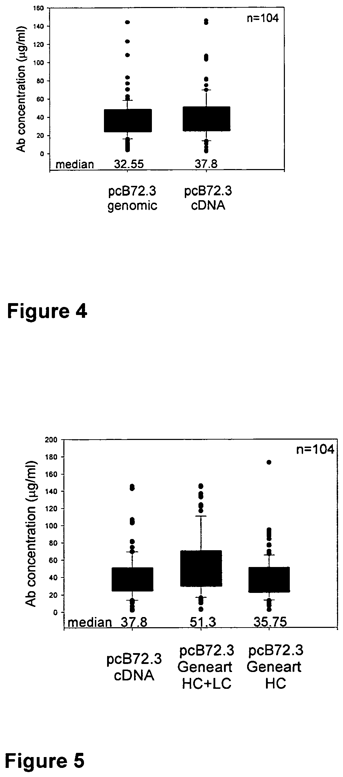

The effect of removing the introns from the HC encoding sequence of pcB72.3 was assessed in stable transfections. 104 colonies of each set of transfectants were allowed to overgrow in 24-well plates, and the resultant antibody concentrations from each cell line determined. The results are summarised in Figure 4 and Table 1. From the data it is evident that the presence of introns in the pcB72.3 HC encoding sequence has no effect on the level of antibody generated. It has been shown that the intron within the promoter sequence is required for maximal gene expression, therefore it is likely that this intron alone contributes to effective gene expression.

There are two possible mechanisms by which this is achieved. Either the promoter itself is more effective when the intron is present, or the mRNA generated following splicing of the promoter derived intron is more stable. It is apparent from this data that additional splicing of introns such as those present in the HC encoding sequence does not result in increased gene expression.

**Table 1: Statistical analysis of data from genomic versus cDNA comparison**

| **Construct** | ***n*** | **Mean (µg/ml)** | **Max (µg/ml)** | **% vs control** | ***p* vs control¹** |
|---|---|---|---|---|---|
| pcB72.3 (control) | 104 | 37.2 | 144 | - | - |
| pcB72.3 HC cDNA | 104 | 41.1 | 145.7 | 110 | 0.686 |
| ⁽¹⁾ ANOVA analysis - values of p ≤ 0.05 are considered significant. | | | | | |

### Analysis of gene-optimized gene sequences

The effect of the DNA sequence optimisation was assessed in stable transfections. 104 colonies of each set of transfectants were allowed to overgrow in 24-well plates, and the resultant antibody concentrations from each cell line determined. In this case, the data was compared to the cDNA version of the control as the gene-optimized sequences were in cDNA format rather than the standard genomic format. The results are summarised in Figure 5 and Table 2.

The gene optimisation demonstrated that optimisation of both chains gave an increase in median antibody concentration of 35% (p=0.006). However, optimisation of the HC alone resulted in no significant increase in antibody expression. These data suggest that also optimisation of the LC is needed for an increased antibody expression. The reason for this might be that the LC sequence was suboptimal and the optimisation has brought the level of LC expressed in line with the level of HC. For example, RNA instability motifs were removed from the LC sequence which were not present in the HC sequence.

**Table 2: Statistical analysis of data from gene optimisations**

| **Construct** | ***n*** | **Mean (µg/ml)** | **Max (µg/ml)** | **% vs control** | ***p vs* control¹** |
|---|---|---|---|---|---|
| pcB72.3 HC cDNA (control) | 104 | 41 | 145.7 | - | - |
| pcB72.3 Geneart HC | 104 | 40.8 | 94.1 | 99.5 | 0.539 |
| pcB72.3 Geneart HC/LC | 104 | 55.4 | 134 | 135 | 0.006 |
| ⁽¹⁾ ANOVA analysis - values of p ≤ 0.05 are considered significant. | | | | | |

### Effect of gene optimized constant regions on antibody production

Vectors were generated that contain the heavy and light chain genes for IgG1, IgG2 and IgG4, respectively, wherein the constant regions of the heavy and light chain genes were gene optimized, whereas the variable regions of both genes were not optimized. Thus, the vector pConG1GA containing the IgG1 gene sequences with optimized constant regions and non-optimized variable regions, the vector pConG2GA containing the IgG2 gene sequences with optimized constant regions and non-optimized variable regions and the vector pConG4GA containing the IgG4 gene sequences with optimized constant regions and non-optimized variable regions were generated.

The vectors obtained were tested in the standard way for antibody production and compared to non-optimized cDNA vectors, in particular the vector G1cDNA containing non-optimized IgG1 heavy and light genes, the vector G2cDNA containing non-optimized IgG2 heavy and light genes and the vector pcB72.3 HCcDNA containing non-optimized IgG4 sequences. Also the fully optimized vector pcB72.3 GAHC/LC, a vector with gene-optimized constant regions and gene optimized variable regions, was included as a control in the first experiment. The results are summarized in Figure 6. A comparison between vectors pConG1GA and G1cDNA shows that the IgG1 optimized constant region gives increased expression. Also a direct comparison between vectors pConG2GA and G2cDNA shows that the IgG2 optimized constant region gives increased expression. In contrast, the IgG4 optimization does not give an increased expression as shown by a comparison between vectors pConG4GA and pcB72.3 HCcDNA. Thus, the results show that using the gene-optimized constant regions results in increased mean antibody expression.

It should be noted that the IgG4 data was obtained from a separate experiment, explaining the differences in the control values observed between the two sets of transfections.

## Claims

1. A mammalian expression vector for Chinese Hamster Ovary host cells comprising at least a first transcription unit containing a first nucleotide sequence encoding the light chain of an antibody and a second transcription unit containing a second nucleotide sequence encoding the heavy chain of an antibody, **characterized in that** the codon composition of both the first and second nucleotide sequence is adapted to the codon bias of Chinese Hamster Ovary host cells.

2. A mammalian expression vector according to claim 1, wherein the light and heavy chains encoded by the first and second nucleotide sequences have amended glycosylation sites.

3. A mammalian expression vector according to claim 1 or claim 2, wherein the light chain and the heavy chain can form an antibody.

4. A mammalian expression vector according to any of claims 1 to 3, wherein the vector comprises a third transcription unit encoding a selectable marker.

5. A mammalian expression vector according to claim 4, wherein the selectable marker is a glutamine synthetase (GS) marker.

6. A Chinese Hamster Ovary host cell containing a mammalian expression vector according to any one of claims 1 to 5.

7. Process for improving the level of production of a secreted antibody comprising a light chain and a heavy chain comprising the steps of
a) transfecting a Chinese Hamster Ovary host cell with an expression vector according to any one of claims 1 to 5 encoding the light chain and the heavy chain,
b) culturing the host cell under appropriate conditions to enable propagation of the cell and expression and assembly of the two chains within the cell to form a molecule and secretion of the molecule formed, and
c) harvesting the antibody formed.

## Patentansprüche

1. Säugetier-Expressionsvektor für Wirtszellen aus dem Eierstock eines Chinesischen Hamsters (CHO-Wirtszellen), umfassend mindestens eine erste Transkriptionseinheit, die eine erste Nukleotidsequenz enthält, die die leichte Kette eines Antikörpers kodiert, und eine zweite Transkriptionseinheit, die eine zweite Nukleotidsequenz enthält, die die schwere Kette eines Antikörpers kodiert, **dadurch gekennzeichnet, dass** die Codon-Zusammensetzung sowohl der ersten als auch der zweiten Nukleotidsequenz an die Codon-Bias von CHO-Wirtszellen angepasst ist.

2. Säuger-Expressionsvektor nach Anspruch 1, wobei die von der ersten und zweiten Nukleotidsequenz kodierten leichten und schweren Ketten geänderte Glykosylierungsstellen aufweisen.

3. Säugetier-Expressionsvektor nach Anspruch 1 oder Anspruch 2, wobei die leichte Kette und die schwere Kette einen Antikörper bilden können.

4. Säugetier-Expressionsvektor nach einem der Ansprüche 1 bis 3, wobei der Vektor eine dritte Transkriptionseinheit umfasst, die einen selektierbaren Marker kodiert.

5. Säugetier-Expressionsvektor nach Anspruch 4, wobei der selektierbare Marker ein Glutaminsynthetase (GS)-Marker ist.

6. Eine CHO-Wirtszelle, die einen Säugetier-Expressionsvektor nach einem der Ansprüche 1 bis 5 enthält.

7. Verfahren zur Verbesserung des Produktionsniveaus eines sezernierten Antikörpers, der eine leichte Kette und eine schwere Kette umfasst, umfassend die Schritte
a) Transfektion einer CHO-Wirtszelle mit einem Expressionsvektor nach einem der Ansprüche 1 bis 5, der die leichte Kette und die schwere Kette kodiert,
b) Kultivierung der Wirtszelle unter geeigneten Bedingungen, um die Vermehrung der Zelle sowie die Expression und Assemblierung der beiden Ketten innerhalb der Zelle zur Bildung eines Moleküls und die Sekretion des gebildeten Moleküls zu ermöglichen, und
c) Ernten des gebildeten Antikörpers.

## Revendications

1. Vecteur d'expression de mammifère pour des cellules hôtes d'ovaire de hamster chinois (cellules hôtes CHO) comprenant au moins une première unité de transcription contenant une première séquence nucléotidique codant pour la chaîne légère d'un anticorps et une seconde unité de transcription contenant une seconde séquence nucléotidique codant pour la chaîne lourde d'un anticorps, **caractérisé en ce que** la composition des codons des première et seconde séquences nucléotidiques est adaptée au biais des codons des cellules hôtes CHO.

2. Vecteur d'expression de mammifère selon la revendication 1, dans lequel les chaînes légères et lourdes codées par les première et seconde séquences nucléotidiques ont des sites de glycosylation modifiés.

3. Vecteur d'expression de mammifère selon la revendication 1 ou la revendication 2, dans lequel la chaîne légère et la chaîne lourde peuvent former un anticorps.

4. Vecteur d'expression de mammifère selon l'une quelconque des revendications 1 à 3, dans lequel le vecteur comprend une troisième unité de transcription codant pour un marqueur sélectionnable.

5. Vecteur d'expression de mammifère selon la revendication 4, dans lequel le marqueur sélectionnable est un marqueur de la glutamine synthétase (GS).

6. Cellule hôte CHO contenant un vecteur d'expression de mammifère selon l'une des revendications 1 à 5.

7. Procédé pour améliorer le niveau de production d'un anticorps sécrété comprenant une chaîne légère et une chaîne lourde comprenant les étapes suivantes
a) la transfection d'une cellule hôte CHO avec un vecteur d'expression selon l'une des revendications 1 à 5 codant pour la chaîne légère et la chaîne lourde,
b) la culture de la cellule hôte dans des conditions appropriées pour permettre la propagation de la cellule ainsi que l'expression et l'assemblage des deux chaînes à l'intérieur de la cellule pour former une molécule et la sécrétion de la molécule formée, et
c) la récolte de l'anticorps formé.


### SEQUENCE LISTING

<110> Lonza Biologics plc.
<120> High-level expression of recombinant antibody in a mammalian host cell
<130> LBP1009PCT00
<160> 4
<170> Patentin version 3.3
<210> 1
   <211> 1500
   <212> DNA
   <213> Homo sapiens
<400> 1
   taaaacgacg gccagtgagc gcgcgtaata cgactcacta tagggcgaat tgggtaccaa      60
   gcttgccgcc accatggagt ggagctgggt gttcctgttc ttcctgagcg tgaccaccgg     120
   cgtgcacagc caggtgcagc tgcagcagag cgacgccgag ctggtgaagc ctggcgccag     180
   cgtgaagatc agctgcaagg ccagcggcta caccttcacc gatcacgcca tccactgggc     240
   caagcagaag cccgagcagg gcctggagtg gatcggctac atcagccccg gcaacgacga     300
   catcaagtac aacgagaagt tcaagggcaa ggccaccctg accgccgaca agagcagcag     360
   caccgcctac atgcagctga acagcctgac cagcgaggac agcgccgtgt acttctgcaa     420
   gcggagctac tacggccact ggggccaggg caccaccctg acagtgagca gcgccagcac     480
   caagggccca agcgtgttcc ccctggcccc ctgcagcaga agcaccagcg agagcacagc     540
   cgccctgggc tgcctggtga aggactactt ccccgagccc gtgaccgtgt cctggaacag     600
   cggagccctg acaagcggag tgcacacctt ccccgccgtg ctgcagagca gcggcctgta     660
   ctccctgagc agcgtggtga ccgtgcccag cagcagcctg ggcaccaaga cctacacctg     720
   caacgtggac cacaagccca gcaacaccaa agtggacaag cgcgtggaga gcaagtacgg     780
   ccctccctgc cccagctgcc ctgcccccga gttcctgggc ggacccagcg tgtttctgtt     840
   cccccccaag cccaaggata ccctgatgat cagccggacc cctgaagtga cctgcgtggt     900
   ggtggatgtg agccaggagg accccgaagt gcagttcaac tggtacgtgg acggcgtgga     960
   agtgcacaac gccaagacca agcccagaga ggagcagttc aacagcacct accgcgtggt    1020
   gtctgtgctg accgtgctgc accaggattg gctgaacggc aaggagtaca agtgcaaagt    1080
   gagcaacaag ggcctgccta gcagcatcga gaaaaccatc agcaaggcca agggccagcc    1140
   aagagagccc caggtgtaca ccctgccccc ctcccaggag gagatgacca agaaccaggt    1200
   gtccctgacc tgtctggtga agggcttcta ccccagcgac atcgccgtgg agtgggagag    1260
   caacggccag cccgagaaca actacaagac caccccccct gtgctggaca gcgatggcag    1320
   cttcttcctg tacagccggc tgaccgtgga taagagcaga tggcaggagg gcaacgtgtt    1380
   cagctgcagc gtgatgcacg aggccctgca caatcactac acccagaaga gcctgagcct    1440
   gtccctgggc aagtgagaat tccgagctcc agcttttgtt ccctttagtg agggttaatt    1500
<210> 2
   <211> 460
   <212> PRT
   <213> Homo sapiens
<400> 2
   Met Glu Trp Ser Trp Val Phe Leu Phe Phe Leu Ser Val Thr Thr Gly
   1               5                   10                  15
   Val His Ser Gln Val Gln Leu Gln Gln Ser Asp Ala Glu Leu Val Lys
               20                  25                  30
   Pro Gly Ala Ser Val Lys Ile Ser Cys Lys Ala Ser Gly Tyr Thr Phe
           35                  40                  45
   Thr Asp His Ala Ile His Trp Ala Lys Gln Lys Pro Glu Gln Gly Leu
       50                  55                  60
   Glu Trp Ile Gly Tyr Ile Ser Pro Gly Asn Asp Asp Ile Lys Tyr Asn
   65                  70                  75                  80
   Glu Lys Phe Lys Gly Lys Ala Thr Leu Thr Ala Asp Lys Ser Ser Ser
                   85                  90                  95
   Thr Ala Tyr Met Gln Leu Asn Ser Leu Thr Ser Glu Asp Ser Ala Val
               100                 105                 110
   Tyr Phe Cys Lys Arg Ser Tyr Tyr Gly His Trp Gly Gln Gly Thr Thr
           115                 120                 125
   Leu Thr Val Ser Ser Ala Ser Thr Lys Gly Pro Ser Val Phe Pro Leu
       130                 135                 140
   Ala Pro Cys Ser Arg Ser Thr Ser Glu Ser Thr Ala Ala Leu Gly Cys
   145                 150                 155                 160
   Leu Val Lys Asp Tyr Phe Pro Glu Pro Val Thr Val Ser Trp Asn Ser
                   165                 170                 175
   Gly Ala Leu Thr Ser Gly Val His Thr Phe Pro Ala Val Leu Gln Ser
               180                 185                 190
   Ser Gly Leu Tyr Ser Leu Ser Ser Val Val Thr Val Pro Ser Ser Ser
           195                 200                 205
   Leu Gly Thr Lys Thr Tyr Thr Cys Asn Val Asp His Lys Pro Ser Asn
       210                 215                 220
   Thr Lys Val Asp Lys Arg Val Glu Ser Lys Tyr Gly Pro Pro Cys Pro
   225                 230                 235                 240
   Ser Cys Pro Ala Pro Glu Phe Leu Gly Gly Pro Ser Val Phe Leu Phe
   245 250 255
   Pro Pro Lys Pro Lys Asp Thr Leu Met Ile Ser Arg Thr Pro Glu Val
   260 265 270
   Thr Cys Val Val Val Asp Val Ser Gln Glu Asp Pro Glu Val Gln Phe
   275 280 285
   Asn Trp Tyr Val Asp Gly Val Glu Val His Asn Ala Lys Thr Lys Pro
   290 295 300
   Arg Glu Glu Gln Phe Asn Ser Thr Tyr Arg Val Val Ser Val Leu Thr
   305 310 315 320
   Val Leu His Gln Asp Trp Leu Asn Gly Lys Glu Tyr Lys Cys Lys Val
   325 330 335
   Ser Asn Lys Gly Leu Pro Ser Ser Ile Glu Lys Thr Ile Ser Lys Ala
   340 345 350
   Lys Gly Gln Pro Arg Glu Pro Gln Val Tyr Thr Leu Pro Pro Ser Gln
   355 360 365
   Glu Glu Met Thr Lys Asn Gln Val Ser Leu Thr Cys Leu Val Lys Gly
   370 375 380
   Phe Tyr Pro Ser Asp Ile Ala Val Glu Trp Glu Ser Asn Gly Gln Pro
   385 390 395 400
   Glu Asn Asn Tyr Lys Thr Thr Pro Pro Val Leu Asp Ser Asp Gly Ser
   405 410 415
   Phe Phe Leu Tyr Ser Arg Leu Thr Val Asp Lys Ser Arg Trp Gln Glu
   420 425 430
   Gly Asn Val Phe Ser Cys Ser Val Met His Glu Ala Leu His Asn His
   435 440 445
   Tyr Thr Gln Lys Ser Leu Ser Leu Ser Leu Gly Lys
   450 455 460
<210> 3
   <211> 800
   <212> DNA
   <213> Homo sapiens
<400> 3
   taaaacgacg gccagtgagc gcgcgtaata cgactcacta tagggcgaat tgggtaccaa 60
   gcttgccgcc accatgagcg tgcccaccca ggtgctgggc ctgctgctgc tgtggctgac 120
   cgatgccaga tgcgacatcc agatgaccca gagccccgcc agcctgagcg tgtctgtggg 180
   cgagaccgtg accatcacct gcagagccag cgagaacatc tacagcaacc tggcctggta 240
   tcagcagaag cagggcaaga gcccccagct gctggtgtac gccgccacca acctggccga 300
   cggcgtgccc agcagattca gcggcagcgg ctccggcacc cagtacagcc tgaagatcaa 360
   cagcctgcag agcgaggatt tcggcagcta ctactgccag cacttctggg gcacccccta 420
   cacctttggc ggcggaacca ggctggagat caagcggacc gtggccgccc ccagcgtgtt 480
   catcttcccc cccagcgatg agcagctgaa gagcggcacc gccagcgtgg tgtgtctgct 540
   gaacaacttc taccctcgag aggccaaagt gcagtggaaa gtggacaacg ccctgcagtc 600
   cggcaacagc caggagagcg tgaccgagca ggacagcaag gactccacct acagcctgag 660
   cagcaccctg accctgagca aggccgacta cgagaagcac aaagtgtacg cctgcgaagt 720
   gacccaccag ggcctgtcca gccccgtgac caagagcttc aaccggggcg agtgctgaga 780
   attccgagct ccagcttttg 800
<210> 4
   <211> 234
   <212> PRT
   <213> Homo sapiens
<400> 4
   Met Ser Val Pro Thr Gln Val Leu Gly Leu Leu Leu Leu Trp Leu Thr
   1 5 10 15
   Asp Ala Arg Cys Asp Ile Gln Met Thr Gln Ser Pro Ala Ser Leu Ser
   20 25 30
   Val Ser Val Gly Glu Thr Val Thr Ile Thr Cys Arg Ala Ser Glu Asn
   35 40 45
   Ile Tyr Ser Asn Leu Ala Trp Tyr Gln Gln Lys Gln Gly Lys Ser Pro
   50 55 60
   Gln Leu Leu Val Tyr Ala Ala Thr Asn Leu Ala Asp Gly Val Pro Ser
   65 70 75 80
   Arg Phe Ser Gly Ser Gly Ser Gly Thr Gln Tyr Ser Leu Lys Ile Asn
   85 90 95
   Ser Leu Gln Ser Glu Asp Phe Gly Ser Tyr Tyr Cys Gln His Phe Trp
   100 105 110
   Gly Thr Pro Tyr Thr Phe Gly Gly Gly Thr Arg Leu Glu Ile Lys Arg
   115 120 125
   Thr Val Ala Ala Pro Ser Val Phe Ile Phe Pro Pro Ser Asp Glu Gln
   130 135 140
   Leu Lys Ser Gly Thr Ala Ser Val Val Cys Leu Leu Asn Asn Phe Tyr
   145                 150                 155                 160
   Pro Arg Glu Ala Lys Val Gln Trp Lys Val Asp Asn Ala Leu Gln Ser
                   165                 170                 175
   Gly Asn Ser Gln Glu Ser Val Thr Glu Gln Asp Ser Lys Asp Ser Thr
               180                 185                 190
   Tyr Ser Leu Ser Ser Thr Leu Thr Leu Ser Lys Ala Asp Tyr Glu Lys
           195                 200                 205
   His Lys Val Tyr Ala Cys Glu Val Thr His Gln Gly Leu Ser Ser Pro
       210                 215                 220
   Val Thr Lys Ser Phe Asn Arg Gly Glu Cys
   225                 230